# FASCICULE DE BREVET EUROPEEN

(11) **EP 2 228 088 B1**
(45) Date de publication et mention de la délivrance du brevet: **19.08.2015**
(21) Numéro de dépôt: 10155240.4
(22) Date de dépôt: 02.03.2010
(51) Int. Cl.: A61M 16/12, A61M 16/04

(54) **Dispositif d'assistance respiratoire**
Vorrichtung zur Atmungsunterstützung
Breathing device for assisted ventilation

(30) Priorité: 11.03.2009 FR 0901122; 14.09.2009 FR 0904364
(43) Date de publication de la demande: 15.09.2010
(73) Titulaire: Boussignac, Georges, 92160 Antony (FR)
(72) Inventeur: Boussignac, Georges, 92160 Antony (FR)
(74) Mandataire: Coralis Harle

(56) Documents cités:
- EP-A1- 0 390 684
- EP-A1- 1 977 780
- WO-A1-03/039638
- WO-A1-2007/118973

## Description

La présente invention a pour objet un dispositif pour assistance respiratoire, utilisable sur des patients dont la respiration spontanée est absente ou insuffisante, qu'ils soient placés ou non sous respiration artificielle.

Par le brevet EP-A-0390684, on connaît déjà un dispositif d'assistance respiratoire comportant :
- un tube, qui forme un canal principal et qui est destiné à être relié par son extrémité distale à une voie respiratoire d'un patient pour que ledit canal principal relie, à l'extérieur, le système respiratoire dudit patient ;
   et
- au moins un canal auxiliaire, par exemple ménagé dans la paroi dudit tube, permettant l'injection d'un jet de gaz respirable destiné à la ventilation du patient, ce canal auxiliaire débouchant dans le canal principal en avant de l'extrémité distale de ce dernier.

Dans un tel dispositif, le gaz respirable alimentant le canal auxiliaire est, le plus souvent, de l'oxygène pur. Or, certains patients, dont l'organisme est habitué à un taux élevé de gaz carbonique dans le sang, ne peuvent supporter une ventilation à l'oxygène pur, qui risquerait d'entraîner un malaise cardiaque.

Aussi, pour remédier à cet inconvénient, on a déjà proposé, par le document EP-A-1441791, un tel dispositif d'assistance respiratoire qui comporte en plus, entre l'orifice distal du canal auxiliaire et l'extrémité distale du canal principal, des moyens de communication fluidique commandables aptes à former, en position ouverte, un passage à section variable reliant ledit canal principal à l'ambiance extérieure, de sorte que de l'air extérieur est aspiré à travers les moyens de communication par le jet de gaz respirable du canal principal. L'air ainsi introduit dilue le gaz respirable, qui peut alors être toléré par les patients, dont le cas a été rappelé ci-dessus.

L'aspiration d'air extérieur provient de la dépression engendrée par le jet de gaz respirable dans le canal principal, en aval de l'orifice distal du canal auxiliaire. Or, la dépression créée s'avère, non seulement, peu élevée, mais également très instable. Elle subit, en effet, des variations imprévisibles, difficilement contrôlables, ce qui rend l'aspiration d'air extérieur irrégulière.

Aussi, l'opérateur, qui manipule le dispositif d'assistance respiratoire utilisé sur le patient, peut être contraint d'ajuster en permanence la section variable du passage formé par les moyens de communication (et ainsi l'intensité de l'aspiration) pour que la dilution du gaz respiratoire par l'air reste sensiblement constante dans le canal principal.

WO 03/039683 décrit un dispositif d'assistance respiratoire.

La présente invention a pour objet de remédier à ces inconvénients.

A cette fin, selon l'invention, le dispositif d'assistance respiratoire tubulaire, qui forme un canal principal destiné à être relié par sa portion distale à une voie respiratoire d'un patient pour que ledit canal principal relie à l'extérieur le système respiratoire dudit patient, ledit dispositif comportant :
- au moins un canal auxiliaire relié à une source de gaz respirable pour pouvoir insuffler un jet d'un tel gaz respirable par au moins un orifice distal disposé en avant de l'extrémité distale dudit canal principal ; et
- des moyens de communication fluidique agencés entre ledit orifice distal dudit canal auxiliaire et ladite extrémité distale dudit canal principal, est remarquable :

- en ce que ledit dispositif d'assistance respiratoire comporte :
   ■ des moyens de dérivation aptes à dériver une fraction de volume dudit gaz respirable provenant de ladite source et destiné audit canal auxiliaire, avant son entrée dans ce dernier ; et
   ■ des moyens d'aspiration d'air ambiant entraînés par ladite fraction dérivée de gaz respirable ;
- et en ce que lesdits moyens d'aspiration sont reliés auxdits moyens de communication, de sorte que ces derniers sont aptes à acheminer, dans ledit canal principal, l'air ambiant aspiré mélangé à ladite fraction dérivée de gaz respirable.

Ainsi, grâce à la présente invention, la fraction de gaz respirable dérivée entraîne les moyens d'aspiration, qui créent une aspiration d'air ambiant, celle-ci étant d'autant plus élevée que la fraction de gaz respirable dérivée est plus importante. En outre, la fraction de gaz respirable dérivée n'étant pas sujette à des variations intempestives et imprévisibles (puisque le flux de gaz respirable provenant de la source de gaz est continu et constant), le risque d'obtenir une aspiration d'air extérieur instable et irrégulière est inexistant. De plus, la fraction de gaz respirable dérivée est mélangée à l'air ambiant aspiré, de sorte que le gaz respirable est dilué avant d'arriver dans le canal principal.

On remarquera donc que, dans la présente invention, l'aspiration d'air ambiant ne dépend pas de la dépression interne siégeant au sein du canal principal en aval de l'orifice distal du canal auxiliaire ; elle est créée volontairement par les moyens d'aspiration.

De préférence, le dispositif d'assistance respiratoire comporte des moyens de réglage de ladite fraction de gaz respirable dérivée par lesdits moyens de dérivation, qui sont avantageusement disposés entre lesdits moyens de dérivation et lesdits moyens d'aspiration. Ces moyens de réglage peuvent comporter au moins une vanne.

Ainsi, on peut régler la fraction de gaz respirable dérivée, de manière à adapter l'intensité de l'aspiration engendrée par les moyens d'aspiration (qui sont entraînés par la fraction de gaz dérivée) et donc le volume d'air ambiant aspiré. De cette façon, on peut ajuster la dilution du gaz respirable dérivé.

Il est à noter que l'on peut envisager d'étalonner la vanne des moyens de réglage de manière à pouvoir contrôler précisément le volume d'air ambiant aspiré et, par conséquent, la dilution du gaz respirable dérivé.

De préférence, le dispositif d'assistance respiratoire comporte des moyens de régulation de débit de gaz respirable dilué sortant desdits moyens d'aspiration et destiné à entrer dans ledit canal principal. Ces moyens de régulation de débit sont avantageusement agencés entre lesdits moyens d'aspiration et lesdits moyens de communication fluidique. En outre, les moyens de régulation de débit peuvent comporter au moins une vanne.

Ainsi, il est possible d'ajuster le débit (et donc la quantité) de gaz respirable dilué entrant dans ledit canal principal à travers lesdits moyens de communication.

De même que pour la vanne des moyens de réglage, il est possible d'étalonner la vanne des moyens de régulation de manière à connaître précisément la quantité de gaz respirable dilué introduit dans le canal principal.

De façon avantageuse, lesdits moyens de communication fluidique comportent au moins un orifice de communication qui est ménagé dans la paroi dudit dispositif.

En outre, dans un exemple de réalisation du dispositif d'assistance respiratoire de l'invention tout spécialement destiné à l'introduction de celui-ci, par la bouche, jusqu'à la carène de l'appareil respiratoire d'un patient, ledit canal principal étant formé par un tube souple, lesdits moyens de communication fluidique comportent avantageusement une gaine souple étanche qui enveloppe, au moins sur une partie de sa longueur, ledit tube souple et qui forme une voie périphérique autour dudit tube souple, dans laquelle débouche ledit orifice de communication et par laquelle est apte à circuler, depuis l'extrémité proximale de ladite gaine souple, l'air ambiant aspiré mélangé à ladite fraction dérivée de gaz respirable.

De cette façon, on peut réaliser un balayage du gaz respiratoire pollué se trouvant dans les poumons en le chassant hors de ceux-ci par du gaz respiratoire frais dilué à l'air ambiant, depuis la carène et à travers le canal principal.

En outre, lesdits moyens d'aspiration sont avantageusement montés directement sur ledit tube souple, au voisinage de son extrémité proximale, ce qui facilite la manipulation du dispositif d'assistance respiratoire.

Par ailleurs, dans un mode de réalisation de l'invention, en regard dudit orifice distal dudit canal auxiliaire, sont prévus des moyens de déflexion dudit jet de gaz respirable de ventilation vers l'axe dudit canal principal et en ce que lesdits moyens de communication sont disposés entre lesdits moyens de déflexion et ladite extrémité distale dudit canal principal.

Les figures du dessin annexé feront bien comprendre comment l'invention peut être réalisée. Sur ces figures, des références identiques désignent des éléments semblables.

La figure 1 est une vue schématique et partielle, en coupe axiale agrandie, d'un premier exemple de réalisation du dispositif d'assistance respiratoire de la présente invention.

Les figures 2, 3 et 4 sont des coupes schématiques transversales, respectivement selon les lignes II-II, III-III et IV-IV de la figure 1.

La figure 5 montre une variante du premier exemple de réalisation du dispositif de l'invention de la figure 1.

La figure 6 montre une autre variante du premier exemple de réalisation du dispositif de l'invention de la figure 1.

La figure 7 est une coupe schématique transversale selon la ligne VII-VII de la figure 6.

La figure 8 est une vue schématique, en coupe axiale agrandie, d'un second exemple de réalisation du dispositif d'assistance respiratoire conforme à l'invention.

Les figures 9 et 10 sont des coupes transversales schématiques du dispositif de la figure 8, respectivement selon les lignes IX-IX et X-X.

La figure 11 est une vue schématique, partiellement en coupe axiale, d'un masque d'assistance respiratoire comportant le dispositif de l'invention illustré en figure 8.

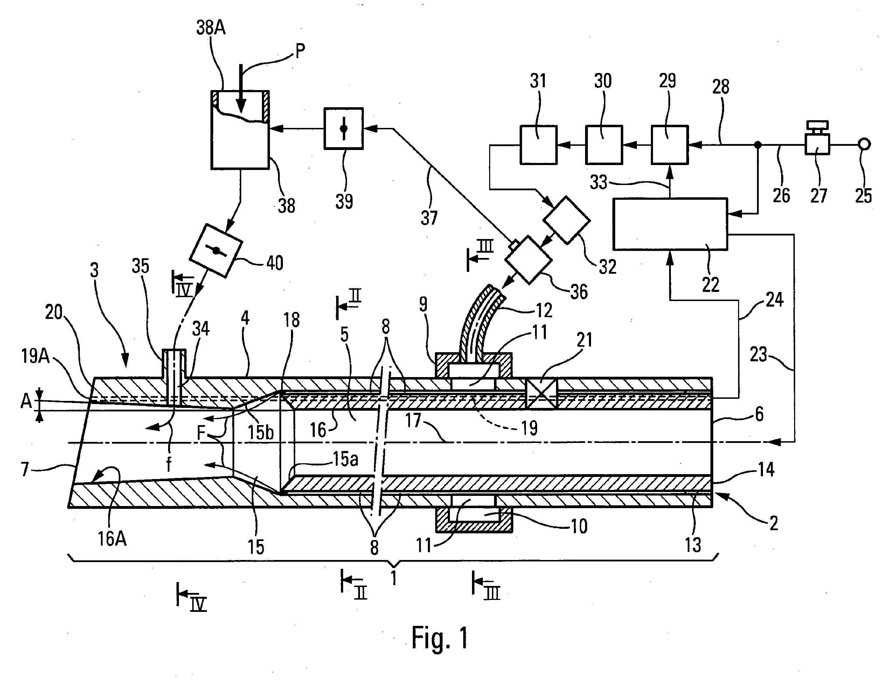

Sur la figure 1, on a représenté, schématiquement et à grande échelle, les seules portions proximale 2 et distale 3 d'un premier exemple de réalisation du dispositif d'assistance respiratoire 1 selon la présente invention. Ce premier exemple de réalisation peut constituer, par exemple, une sonde endotrachéale oro-nasale avec ou sans ballonnet, une sonde endotrachéale pédiatrique, une sonde de monitorage des gaz, une sonde endobronchique, une sonde d'intubation anatomique pour enfant, une sonde de Cole néonatale, une sonde canule de Gedel, une sonde nasale d'oxygénothérapie, un masque nasal ou bucconasal ou un ballon nasal pour traitement d'apnée du sommeil.

Le dispositif 1 comporte un tube 4, souple ou préformé (pour s'adapter à la morphologie du patient) délimitant un canal principal 5 ayant un orifice proximal 6 et un orifice distal 7, respectivement aux extrémités dudit tube 4.

Ainsi, le canal principal 5 est capable d'assurer le passage entre les orifices proximal 6 et distal 7, dont l'un (orifice distal 7) est destiné à se trouver à l'intérieur des voies respiratoires d'un patient et l'autre (orifice proximal 6) est destiné à se trouver à l'extérieur dudit patient. Cet orifice proximal 6 peut déboucher à l'air libre et, dans ce cas, le patient peut inspirer de l'air frais et expirer de l'air vicié à travers le canal principal 5. On peut également, comme cela est expliqué par la suite, relier l'orifice 6 à une source de gaz respirable sous pression et prévoir un système de valves unidirectionnelles, pour que le patient inspire le gaz respirable de ladite source à travers ledit canal principal 5 et expire le gaz vicié à l'air libre, également à travers ce canal principal 5.

Le diamètre du canal principal 5 est de l'ordre de quelques millimètres. Des essais satisfaisants ont été effectués avec des diamètres de 3 mm, 7 mm, 8 mm et 12 mm.

Par ailleurs, dans l'épaisseur de la paroi du tube 4, sont ménagés des canaux auxiliaires 8, s'étendant sur la presque totalité de la longueur du canal principal 5 et destinés à être reliés à une source de gaz respirable sous pression, comme cela est décrit ci-après.

La liaison à la source de gaz respirable peut être réalisée au moyen d'une bague 9, entourant de façon étanche le tube 4, du côté de l'extrémité proximale 2 et délimitant une chambre annulaire étanche 10 autour dudit tube 4. Les canaux auxiliaires 8 sont mis en communication avec la chambre annulaire 10, grâce à des arrachements locaux 11 de la paroi du tube 4, et ladite chambre 10 est reliée à ladite source de gaz respirable par un conduit 12. Bien entendu, les extrémités proximales des canaux 8 sont obturées, par exemple par des bouchons 13, introduits à partir de la face d'extrémité proximale 14 du tube 4.

Les canaux auxiliaires 8 ont un diamètre plus petit que celui du canal principal 5. Le diamètre des canaux auxiliaires 8 est de préférence inférieur à 1 mm et, de façon avantageuse, il est de l'ordre de 5 à 800 microns. Du côté distal, les canaux auxiliaires 8 débouchent dans un évidement 15 de la paroi interne 16 du tube 4. L'évidement 15 est annulaire et centré sur l'axe 17 dudit tube 4. Il comporte une face 15a, sensiblement transversale ou légèrement inclinée de façon à constituer un évasement du canal principal 5, dans laquelle débouchent lesdits canaux auxiliaires 8 par leurs orifices 18, ainsi qu'une face 15b suivant la face 15a et convergeant en direction de l'axe 17.

De préférence, entre la face inclinée convergente 15b et l'orifice distal 7, la paroi interne 16 du tube 4 présente une partie légèrement évasée vers l'extérieur, comme cela est illustré par l'angle A sur la figure 1.

Ainsi, lorsque les canaux auxiliaires 8 sont alimentés en gaz respirable sous pression à travers les éléments 9 à 12, les jets gazeux correspondants heurtent la face inclinée 15b, qui les défléchit en direction de l'axe 17 (flèche F sur la figure 1), engendrant au voisinage de celui-ci une zone de pression favorisant la circulation gazeuse à l'intérieur du canal principal 5, de l'orifice proximal 6 vers l'orifice distal 7. On favorise ainsi l'inspiration du patient.

Au moins un canal supplémentaire 19 est prévu dans l'épaisseur du tube 4 afin de déboucher en 19A au voisinage de la face d'extrémité distale 20 du tube 4 et servir de prise de pression.

A titre de sécurité, une soupape d'échappement tarée 21 peut être prévue au voisinage de la portion proximale 2 du tube 4. Ainsi, en cas de surpression accidentelle dans le canal principal 5, une fuite de gaz se produit à l'extérieur du patient, à travers la paroi du tube 4, pour éliminer instantanément cette surpression.

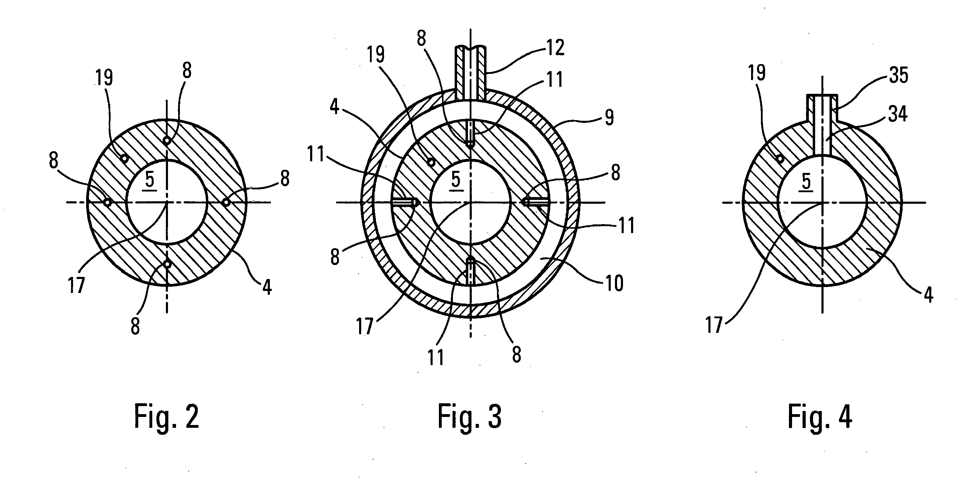

Comme le montrent les figures 2 et 3, les canaux auxiliaires 8 sont disposés régulièrement autour de l'axe du tube 4. Leur nombre est variable suivant les utilisations (adulte ou enfant), mais il est généralement compris entre trois et neuf. De plus, au moins un des canaux auxiliaires 8 peut être spécialisé pour apporter un fluide médical.

Le tube 4 du dispositif 1 selon l'invention peut être réalisé en toute matière déjà utilisée dans les sondes respiratoires, par exemple en un chlorure de polyvinyle, avec un éventuel revêtement en silicone ou en acier permettant les injections à pression élevée.

Bien entendu, les dimensions du dispositif 1 selon l'invention peuvent être très variables, essentiellement en fonction de la voie de mise en place du tube et de la taille du patient, qui peut être un adulte, un enfant, un nourrisson ou un prématuré.

Le dispositif 1 comporte de plus un dispositif d'alimentation et de commande 22, qui est respectivement relié à l'orifice proximal 6 du tube 4 par une liaison 23 et au canal supplémentaire 19 par une liaison 24.

Le dispositif d'alimentation et de commande 22 est alimenté en gaz respirable sous pression, par exemple de l'oxygène pur, par une source 25, à laquelle il est relié par une conduite 26 sur laquelle est monté un détenteur-débitmètre réglable 27.

La sortie du détenteur-débitmètre 27 est reliée au conduit 12 par une conduite de dérivation 28 sur laquelle sont montés en série une vanne commandable 29, un dispositif à perte de charge réglable limiteur de débit et de pression 30 (par exemple un tube à conduit calibré), un humidificateur 31 et une soupape d'échappement tarée 32, à tarage réglable. La vanne commandable 29 est commandée par le dispositif d'alimentation et de commande 22 par l'intermédiaire d'une liaison 33.

A titre d'exemple non limitatif, le détenteur-débitmètre 27 peut délivrer, dans la conduite 28, le gaz respirable provenant de la source 25 sous une pression P, par exemple égale à 3,5 bars avec un débit maximal réglable de par exemple 32 litres par minute, alors que limiteur de débit et de pression 30, recevant ce gaz respirable de la conduite 28, peut en abaisser la pression jusqu'à une valeur p, égale par exemple à 0,5 bar pour un adulte et à 0,07 bar pour un enfant, et le débit jusqu'à une valeur d égale, par exemple, à 0,5 litre par minute. Quant à elle, la soupape d'échappement 32 est tarée à la pression p.

Par ailleurs (voir les figures 1 et 4), entre l'évidement annulaire 15 et l'orifice distal 7, la paroi du tube 4 est percée par un orifice de communication 34 prolongé radialement à l'extérieur du tube 4 par un embout d'entrée de gaz 35, l'orifice 34 et l'embout d'entrée 35 formant des moyens de communication fluidique au dispositif 1.

Comme le montre la figure 1, entre la soupape d'échappement 32 et la bague 9 du tube 4, des moyens 36 de dérivation d'une fraction de volume du gaz respirable (provenant de la source 25) sont montés sur le conduit 12. Les moyens de dérivation 36 (représentés schématiquement par un carré sur la figure 1) peuvent, par exemple, comporter un élément de dérivation en forme de T ou de Y. Bien entendu, on peut également envisager de disposer différemment les moyens de dérivation, par exemple en les agençant sur la conduite de dérivation 28.

La sortie dérivée des moyens de dérivation 36 du conduit 12 est reliée à l'embout d'entrée de gaz 35 par un conduit de dérivation 37 sur lequel sont montés, entre les moyens de dérivation 36 et l'embout d'entrée 35, des moyens 38 d'aspiration d'air ambiant.

Ces moyens d'aspiration 38, fonctionnant par exemple sur un principe semblable à celui d'une pompe à entrainement, sont entraînés par la fraction de gaz respirable dérivée du conduit 12 et provenant de la source 25. Ils sont ainsi aptes à pomper de l'air ambiant (symbolisé par la flèche P) par un orifice d'entrée d'air 38A. En sortie des moyens d'aspiration 38, le gaz respirable dérivé est dilué par l'air ambiant aspiré.

Comme le montre la figure 1, entre les moyens de dérivation 36 et les moyens d'aspiration 38, une vanne de réglage 39, apte à régler la fraction de gaz respirable dérivée, est montée sur le conduit de dérivation 37. Ainsi, en adaptant la fraction dérivée de gaz respirable, la vanne de réglage 39 permet d'ajuster l'intensité de l'aspiration engendrée par les moyens d'aspiration 38 (qui sont entraînés par ladite fraction dérivée) et donc le volume d'air ambiant aspiré.

Avantageusement, on peut envisager d'étalonner la vanne de réglage 39, de manière à pouvoir contrôler précisément le volume d'air ambiant aspiré et, par conséquent, la dilution du gaz respirable dérivé.

Tel qu'illustré sur la figure 1, une vanne de régulation 40 peut également être montée sur le conduit de dérivation 37, entre les moyens d'aspiration 38 et l'embout d'entrée de gaz 35 du tube 4. Cette vanne de régulation 40 est, quant à elle, apte à réguler le débit de gaz respirable dilué entrant dans le canal principal 5 (voir flèche f), par l'orifice 34.

Les modes de fonctionnement du dispositif 1 conforme au premier exemple de réalisation (figures 1 à 4) sont les suivants :
- dans le mode de respiration artificielle, les vannes de réglage 39 et de régulation 40 sont fermées et le dispositif d'alimentation et de commande 22, d'une part, commande la vanne commandable 29 à la fermeture par l'intermédiaire de la liaison 33, de sorte que le conduit 12 n'est pas alimenté en gaz respirable et, d'autre part, adresse du gaz respirable dans le tube 4 par l'intermédiaire de la liaison 23. Ce dispositif 22 comporte des moyens (non représentés) pour permettre le réglage de la pression et du débit du gaz respirable qu'il reçoit de la conduite 26 et qu'il adresse au tube 4. Si une surpression se produit dans la voie respiratoire du patient, elle est détectée et transmise, par le canal supplémentaire 19 et la liaison 24, au dispositif 22 qui arrête son fonctionnement. De plus, si cette surpression dépasse le seuil de tarage de la soupape tarée 21 -par exemple du fait que le canal supplémentaire 19 est obstrué par des mucosités et n'a pas pu transmettre l'information de surpression au dispositif 22- cette soupape 21 s'ouvre et le canal principal 5 est mis à l'atmosphère ;
- dans le mode d'assistance respiratoire, le dispositif d'alimentation et de commande 22 coupe la liaison 23, pour mettre l'orifice proximal 6 en communication avec l'atmosphère, et commande la vanne commandable 29 par la liaison 33 pour que celle-ci adresse au patient un jet, continuel ou impulsionnel, de gaz respirable à travers le limiteur 30, l'humidificateur 31, la soupape d'échappement tarée 32, les moyens de dérivation 36 et les canaux auxiliaires 8. Par ailleurs, les vannes de réglage 39 et de régulation 40 sont ouvertes. Par suite, de l'air ambiant est aspiré par les moyens d'aspiration 38 (voir flèche P) et mélangé avec le gaz respirable dérivé (par les moyens de dérivation 36 du conduit 12) qui est ainsi dilué. Bien entendu, le taux de dilution du gaz respirable dérivé dépend de l'aspiration d'air ambiant des moyens d'aspiration 38 et donc de l'ouverture de la vanne de réglage 39. On peut remarquer que, pour des conditions constantes d'injection de gaz respirable dans le conduit 12, le taux de dilution correspondant à l'ouverture de la vanne de réglage 39 peut être étalonné une fois pour toutes, de sorte que l'on peut délivrer à un patient le mélange air-gaz respirable le plus approprié à son cas en choisissant une ouverture donnée de cette vanne de réglage 39. En outre, le débit de gaz respirable dilué entrant dans le canal principal via l'orifice 34 peut être contrôlé précisément par l'intermédiaire de la vanne de régulation 40. Si une surpression se produit dans la voie respiratoire du patient, comme cela a été décrit ci-dessus, cette surpression est détectée et transmise par le canal supplémentaire 19, de sorte que le dispositif 22 ferme la vanne commandable 29 et que la conduite 28 cesse d'adresser du gaz au patient. Si le canal supplémentaire 19 est obstrué, le dispositif 22 n'est pas averti de la surpression dans la voie respiratoire du patient et ne peut s'arrêter, mais cette surpression entraîne une augmentation de pression dans les canaux auxiliaires 8 et le conduit 12. Lorsque cette augmentation de pression atteint le seuil d'ouverture de la soupape de sécurité 32, celle-ci s'ouvre et le jet de gaz respirable n'est plus adressé au patient, mais au contraire est dérivé vers l'extérieur par ladite soupape de sécurité 32. Ainsi, bien que dans ce dernier cas la sécurité 19A, 19, 24, 22, 29 n'ait pu fonctionner, le jet de gaz respirable ne peut atteindre le système respiratoire du patient.

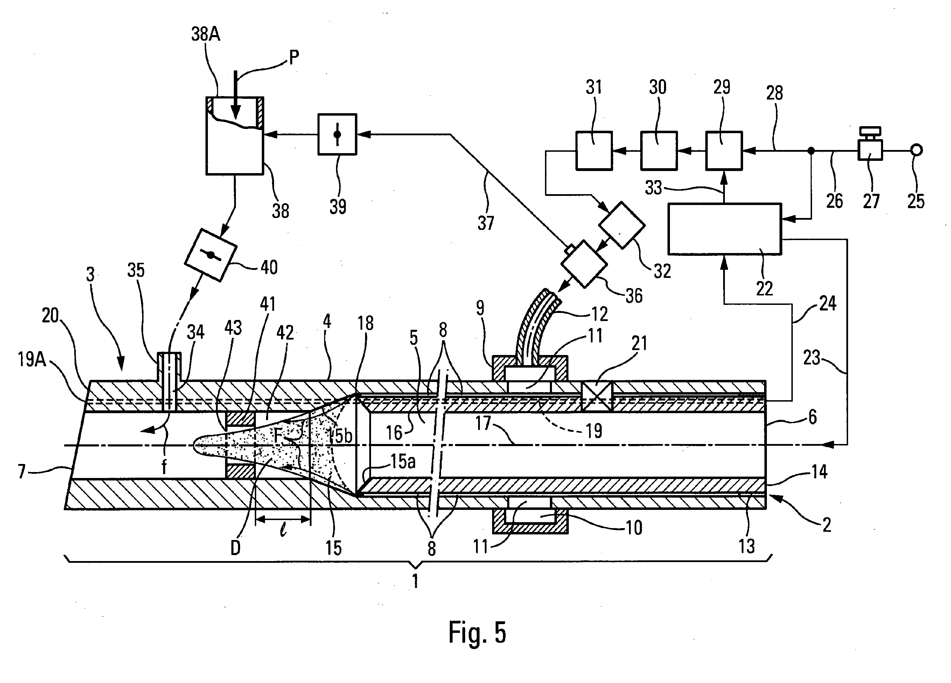

Dans une variante du premier exemple de réalisation illustrée en figure 5 (dont le fonctionnement est identique à celui décrit ci-dessus), une bague aval 41 est disposée dans la portion distale 3 du tube 4, entre l'évidement annulaire 15 et l'orifice 34 des moyens de communication fluidique. Cette bague 41 entoure la zone de pression centrale du canal principal 5 (référencée D sur la figure 5) et occupe localement au moins en partie l'espace périphérique annulaire 42 compris entre ladite zone de pression centrale D et la paroi interne 16 de la portion distale 3 du canal principal 5.

Grâce à une telle bague 41, la pression de la source de gaz respirable 25, nécessaire à l'obtention de la zone de pression D oblongue, peut être abaissée tout en obtenant une zone de pression D à pression identique.

En règle générale, la distance ℓ entre la bague 41 et la face inclinée de déflexion 15b est voisine du diamètre de la partie distale du canal principal 5.

Toutefois, il peut être avantageux, pour permettre d'obtenir la réduction optimale de pression nécessaire de la source 25, que cette distance ℓ puisse être réglable. Il est également avantageux, à la même fin, que le diamètre de l'ouverture centrale 43 de la bague 41 soit réglable.

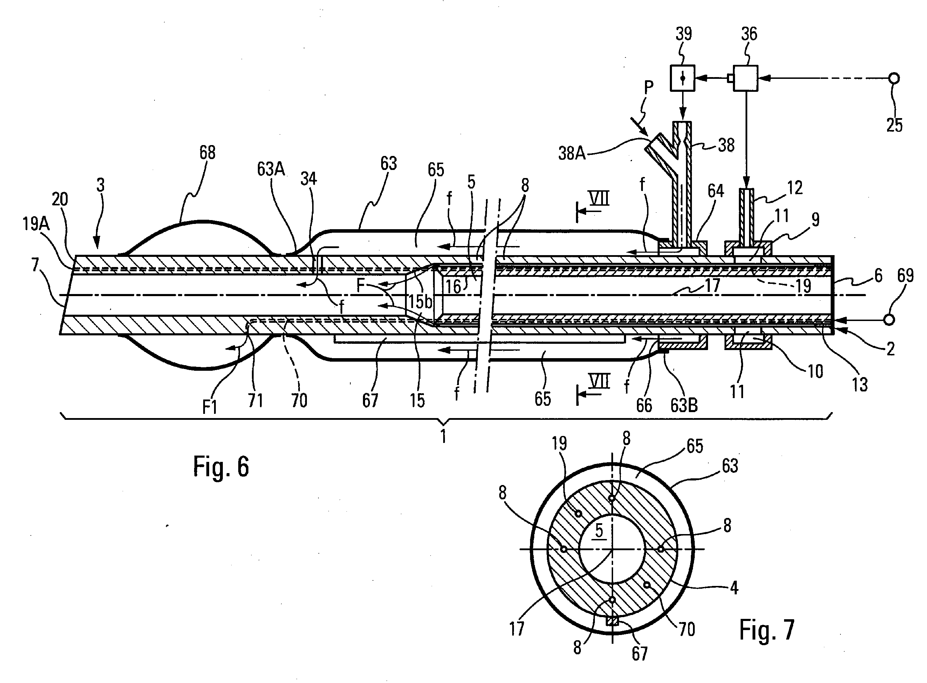

Sur la figure 6, on a illustré une autre variante du premier exemple de réalisation du dispositif 1 destinée à l'introduction de celui-ci, par la bouche, jusqu'à la carène de l'appareil respiratoire d'un patient.

Comme le montre la figure 6, le tube souple 4 est enveloppé, sur la plus grande partie de sa longueur, d'une gaine souple étanche 63. L'extrémité distale 63A de la gaine souple 63 peut être solidarisée, de façon étanche, au voisinage de l'extrémité distale 7 du tube 4 et ce dernier comporte, entre l'évidement annulaire 15 et l'orifice distal 7, un orifice de communication 34 disposé à l'intérieur de ladite gaine souple 63. L'extrémité proximale 63B de celle-ci peut, quant à elle, être solidarisée, de façon étanche, à un embout annulaire 64, disposé du côté proximal 2 du tube souple 4 et relié aux moyens d'aspiration 38. De cette façon, la gaine souple 63 forme une voie périphérique 65 autour du tube souple 4 qui débouche dans le canal principal 5 par l'intermédiaire de l'orifice 34.

Une ouverture annulaire 66, pratiquée dans l'embout annulaire 64, permet de mettre en communication la gaine souple 63 avec le gaz respirable frais dilué par l'air ambiant. Ainsi, le gaz respirable frais dilué, provenant des moyens 38, peut circuler dans la voie périphérique 65 et entrer dans le canal principal 5 (flèche f), par les orifices 34.

Dans cette autre variante, les moyens d'aspiration 38, sous la forme d'un tube de Venturi, sont montés sur l'embout annulaire 64 du tube souple 4, afin de faciliter la manipulation du dispositif 1.

Par ailleurs, comme le montrent les figures 6 et 7, la paroi externe du tube 4, recouverte par la gaine souple 63, comporte une nervure longitudinale saillante 67 destinée à empêcher une obturation hermétique de la voie périphérique 65. En variante, on peut pratiquer une rainure dans la paroi externe du tube 4, en lieu et place de ladite nervure 67.

De plus, de façon connue, le tube souple 4 peut comporter un ballon de fixation 68 gonflable, en aval de la gaine souple 63. Le ballon gonflable 68, simple ou double, peut être gonflé par un gaz de gonflage (flèche F1) provenant d'une source 69 et acheminé par un canal supplémentaire 70, ménagé dans l'épaisseur du tube 4. Ce canal 70 débouche dans le ballon de fixation 68 en 71. En outre, la membrane du ballon 68 peut être au moins partiellement renforcée de manière à éviter toute déchirure due à une morsure du patient, lors de l'introduction du tube souple 4 par la bouche de celui-ci.

Ainsi, dans cette autre variante du premier exemple, le tube souple 4 peut être aisément introduit dans l'appareil respiratoire d'un patient, jusqu'à ce que son extrémité distale 7 se trouve au voisinage de la carène (non représentée). L'espace mort entre l'extrémité distale 7 du tube souple 4 et les poumons du patient est alors réduit à un minimum.

De cette façon, on peut réaliser un balayage du gaz respiratoire pollué se trouvant dans les poumons en le chassant hors de ceux-ci par du gaz respiratoire frais dilué à l'air ambiant, depuis la carène et à travers le canal principal 5.

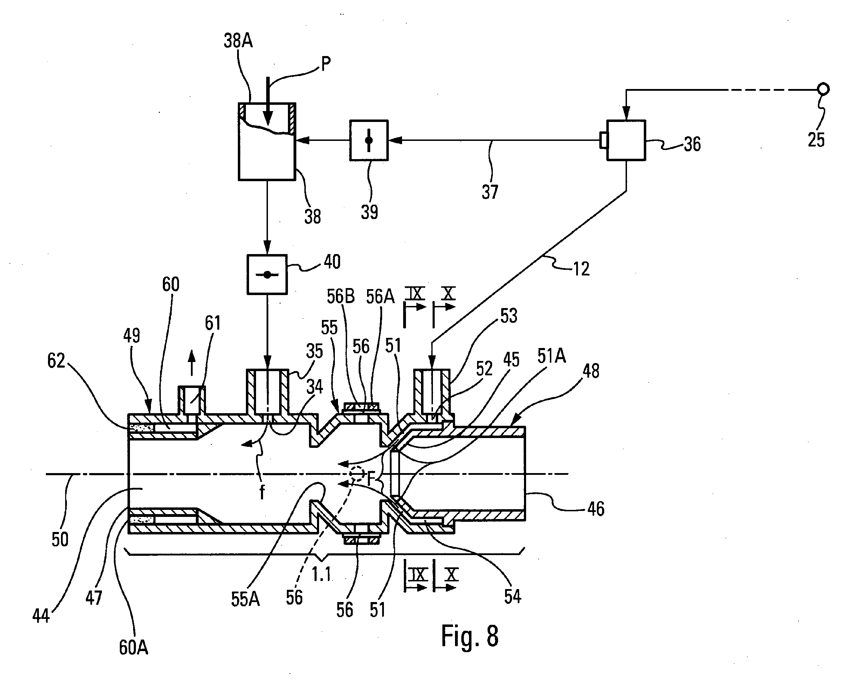

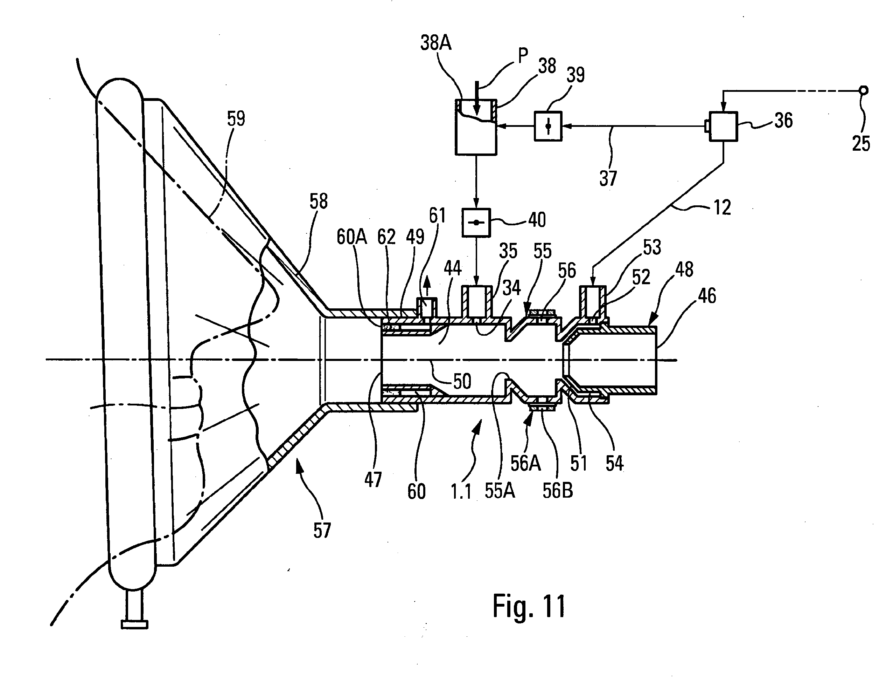

Comme le montrent les figures 8 à 11, le dispositif d'assistance respiratoire conforme au second exemple de réalisation de l'invention est un embout tubulaire 1.1 qui comporte un passage traversant interne 44 et une paroi conique 45 saillant à l'intérieur dudit passage interne 44.

Le passage interne 44 est délimité par un orifice proximal 46 et par un orifice distal 47, respectivement aux extrémités proximale 48 et distale 49 dudit embout tubulaire 1.1.

La paroi conique 45 a pour objet de défléchir, en direction de l'axe longitudinal 50 du passage interne 44, des jets de gaz respirable injectés par des canaux auxiliaires 51, alimentés à partir d'un orifice 52 prolongé par un embout latéral d'amenée 53, par l'intermédiaire d'une chambre annulaire périphérique 54. Les jets de gaz respirable, provenant d'une source de gaz respirable 25, débouchent des canaux auxiliaires 51 par leurs orifices 51 A.

L'embout d'amenée 53 est relié à des moyens de dérivation 36 (semblables à ceux décrits précédemment en relation avec la figure 1) par un conduit 12.

Par ailleurs, au voisinage de sa portion distale 48, la paroi de l'embout tubulaire 1.1 est percée d'un orifice de communication 34 qui est prolongé radialement vers l'extérieur par un embout latéral d'entrée de gaz 35, l'orifice 34 et l'embout 35 formant des moyens de communication fluidique au dispositif 1.1.

Ces moyens de communication fluidique sont aptes à être alimentés en gaz respirable dilué par un conduit de dérivation 37, à travers les moyens de dérivation 36, d'une vanne de réglage 39, de moyens d'aspiration 38 et d'une vanne de régulation 40 (décrits ci-dessus en relation avec la figure 1).

Par ailleurs, l'embout tubulaire 1.1 comporte une portion tubulaire médiane 55, intercalée entre la portion proximale 48 et la portion distale 49, dont l'extrémité longitudinale distale 55A est légèrement saillante à l'intérieur du passage interne 44, de manière à former une bague aval de fonctionnement semblable à celui de la bague aval 41 précitée.

Des orifices d'échappement 56 sont percés dans la paroi latérale de la portion médiane 55, de manière à relier le passage interne 44 à l'air ambiant. De préférence, ces orifices d'échappement 56 sont répartis régulièrement autour de l'axe 50, sur une même section de la portion médiane 55. Ils permettent de faciliter l'expiration du patient en laissant s'échapper du gaz vicié du système respiratoire de ce dernier.

On peut en outre recouvrir les orifices d'échappement 56 par une bague 56A, apte à tourner à frottement doux autour de la portion médiane 55 et elle-même pourvue de trous 56B, de diamètre au moins égal à celui des orifices 56, pouvant être amenés en regard des orifices 56 par rotation de la bague 56A.

Par ailleurs, du côté de la portion distale 49, l'embout tubulaire 1.1 comporte une chambre périphérique annulaire 60, coaxiale audit embout 1.1. La chambre périphérique annulaire 60 débouche au niveau de l'extrémité distale 47 de l'embout 1.1 et est pourvue, à son extrémité proximale, d'un embout de sortie 61, qui peut être relié à un analyseur de gaz et à un dispositif de mesure de pression (non représentés).

Un filtre 62, fibreux ou poreux, est disposé dans la chambre périphérique annulaire 60, pour amortir les turbulences gazeuses et, par suite, les trop fortes variations de pression.

Bien que sur les figures 8 à 10, on ne les ait pas représentés, il va de soi que l'embout tubulaire 1.1 peut comporter des canaux ou conduits d'injection de médicaments et/ou d'eau.

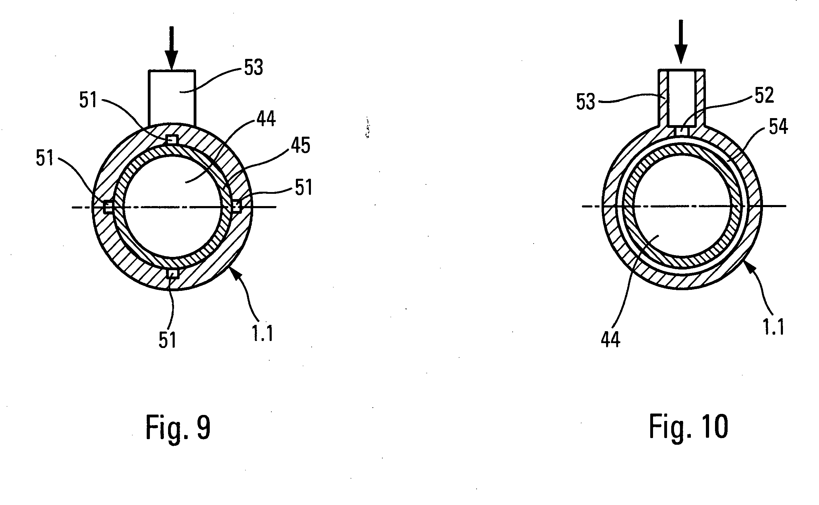

Sur la figure 9, on a représenté un masque d'assistance respiratoire 57 qui comporte une coque rigide 58 de forme générale tronconique, pouvant être appliquée sur le visage d'un patient 59. Du côté opposé, ledit masque 57 comporte le dispositif tubulaire 1.1, conforme au second exemple de réalisation de la présente invention. Ce dispositif tubulaire 1.1 sert d'embout d'entrée et de sortie de gaz dans le masque 57.

Bien entendu, on comprendra aisément que le dispositif 1, 1.1, conforme à la présente invention, peut trouver de nombreuses autres applications, par exemple comme sonde nasale, sonde buccale, sonde trachéale, masque laryngé, King système, COMBITUBE (marque déposée), etc ... Il est évident que les dimensions dudit dispositif sont alors adaptées à chaque utilisation particulière.

## Revendications

1. Dispositif d'assistance respiratoire tubulaire, qui forme un canal principal (5 ; 44) destiné à être relié par sa portion distale (3 ; 49) à une voie respiratoire d'un patient pour que ledit canal principal (5 ; 44) relie à l'extérieur le système respiratoire dudit patient, ledit dispositif (1 ; 1.1) comportant :
- au moins un canal auxiliaire (8 ; 51) relié à une source de gaz respirable (25) pour pouvoir insuffler un jet d'un tel gaz respirable par au moins un orifice distal (18 ; 51 A) disposé en avant de l'extrémité distale (7 ; 47) dudit canal principal (5 ; 44) ; et
- des moyens de communication fluidique agencés entre ledit orifice distal (18 ; 51 A) dudit canal auxiliaire (8 ; 51) et ladite extrémité distale (7 ; 47) dudit canal principal (5 ; 44),
**caractérisé :**
- **en ce que** ledit dispositif d'assistance respiratoire (1 ; 1.1) comporte :
▪ des moyens (36) de dérivation aptes à dériver une fraction de volume dudit gaz respirable destiné audit canal auxiliaire (8 ; 51), avant son entrée dans ce dernier ; et
▪ des moyens (38) d'aspiration d'air ambiant entraînés par ladite fraction dérivée de gaz respirable ;
- et **en ce que** lesdits moyens (38) d'aspiration sont reliés auxdits moyens de communication (34), de sorte que ces derniers sont aptes à acheminer, dans ledit canal principal (5 ; 44), l'air ambiant aspiré mélangé à ladite fraction dérivée de gaz respirable.

2. Dispositif selon la revendication 1,
**caractérisé en ce qu'**il comporte des moyens (39) de réglage de ladite fraction de gaz respirable dérivée par lesdits moyens de dérivation (36).

3. Dispositif selon la revendication 2,
**caractérisé en ce que** lesdits moyens de réglage (39) sont disposés entre lesdits moyens de dérivation (36) et lesdits moyens d'aspiration (38).

4. Dispositif selon l'une des revendications 2 ou 3,
**caractérisé en ce que** lesdits moyens de réglage (39) comportent au moins une vanne.

5. Dispositif selon l'une des revendications 1 à 4,
**caractérisé en ce qu'**il comporte en outre des moyens (40) de régulation de débit de gaz respirable dilué sortant desdits moyens d'aspiration (38) et destiné à entrer dans ledit canal principal (5 ; 44).

6. Dispositif selon la revendication 5,
**caractérisé en ce que** lesdits moyens de régulation de débit (40) sont agencés entre lesdits moyens d'aspiration (36) et lesdits moyens de communication fluidique (34).

7. Dispositif selon l'une des revendications 5 ou 6,
**caractérisé en ce que** lesdits moyens de régulation de débit (40) comportent au moins une vanne.

8. Dispositif selon l'une des revendications 1 à 7,
**caractérisé en ce que** lesdits moyens de communication fluidique comportent au moins un orifice de communication (34) qui est ménagé dans la paroi dudit dispositif (1 ; 1.1).

9. Dispositif selon la revendication 8,
**caractérisé en ce que**, ledit canal principal (5) étant formé par un tube souple (4), lesdits moyens de communication fluidique comportent en outre une gaine souple étanche (63) qui enveloppe, au moins sur une partie de sa longueur, ledit tube souple (4) et qui forme une voie périphérique (65) autour dudit tube souple (4), dans laquelle débouche ledit orifice de communication (34) et par laquelle est apte à circuler, depuis l'extrémité proximale (63B) de ladite gaine souple (63), l'air ambiant aspiré mélangé à ladite fraction dérivée de gaz respirable.

10. Dispositif selon la revendication 9,
**caractérisé en ce que** lesdits moyens d'aspiration (38) sont montés directement sur ledit tube souple (4) au voisinage de son extrémité proximale (2).

11. Dispositif selon l'une des revendications 1 à 10,
**caractérisé en ce que**, en regard dudit orifice distal (18 ; 51 A) dudit canal auxiliaire (8 ; 51), sont prévus des moyens (15 ; 45) de déflexion dudit jet de gaz respirable de ventilation vers l'axe (17 ; 50) dudit canal principal (5 ; 44) et **en ce que** lesdits moyens de communication (34, 35) sont disposés entre lesdits moyens de déflexion (15 ; 45) et ladite extrémité distale (7 ; 47) dudit canal principal (5 ; 44).

## Patentansprüche

1. Rohrförmige Vorrichtung zur Atmungsunterstützung, die einen Hauptkanal (5; 44) bildet, der dazu bestimmt ist, an seinem distalen Teil (3; 49) mit einer Luftröhre eines Patienten verbunden zu werden, damit der Hauptkanal (5; 44) das Atmungssystem des Patienten nach aussen hin verbindet, wobei die Vorrichtung (1; 1.1)
- wenigstens einen Hilfskanal (8; 51), der mit einer Quelle von Atemgas (25) verbunden ist, um einen Strahl eines solchen Atemgases durch wenigstens eine vor dem distalen Ende (7; 47) des besagten Hauptkanals (5; 44) angeordnete distale Öffnung (18; 51A) einblasen zu können; und
- Mittel für eine fluidische Verbindung, die zwischen der distalen Öffnung (18; 51A) des Hilfskanals (8; 51) und dem distalen Ende (7; 47) des Hauptkanals (5; 44) angeordnet ist,
aufweist,
**dadurch gekennzeichnet, dass**
- die Vorrichtung zur Atmungsunterstützung (1; 1.1)
= Abzweigmittel (36), die dazu geeignet sind, einen Teil des für den Hilfskanal (8; 51) bestimmten Volumens des Atemgases vor dessen Einfliessen in letzteren abzuzweigen; und
= Mittel (38) zum Ansaugen von Umgebungsluft, die durch die abgezweigte Menge des Atemgases angetrieben werden, aufweist, und
- die Mittel (38) zum Ansaugen mit den Verbindungsmitteln (34) verbunden sind, so dass letztere geeignet sind, angesaugte und mit dem abgezweigten Teil des Atemgases vermischte Umgebungsluft dem Hauptkanal (5; 44) zuzuführen.

2. Vorrichtung gemäss Anspruch 1, **dadurch gekennzeichnet, dass** sie Mittel (39) zum Regeln des durch die Abzweigmittel (36) abgezweigten Teils des Atemgases aufweist.

3. Vorrichtung gemäss Anspruch 2, **dadurch gekennzeichnet, dass** die Regelungsmittel (39) zwischen den Abzweigmitteln (36) und den Ansaugmitteln (38) angeordnet sind.

4. Vorrichtung gemäss einem der Ansprüche 2 oder 3, **dadurch gekennzeichnet, dass** die Regelungsmittel (39) wenigstens ein Ventil aufweisen.

5. Vorrichtung gemäss einem der Ansprüche 1 bis 4, **dadurch gekennzeichnet, dass** sie ausserdem Mittel (40) zum Regeln des Durchsatzes des gemischten Atemgases, das aus den Ansaugmitteln (38) herauskommt und dazu bestimmt ist, in den Hauptkanal (5; 44) einzufliessen, aufweist.

6. Vorrichtung gemäss Anspruch 5, **dadurch gekennzeichnet, dass** die Mittel (40) zum Regeln des Durchsatzes zwischen den Ansaugmitteln (36) und den Mitteln für eine fluidische Verbindung (34) angeordnet sind.

7. Vorrichtung gemäss einem der Ansprüche 5 oder 6, **dadurch gekennzeichnet, dass** die Mittel (40) zum Regeln des Durchsatzes wenigstens ein Ventil aufweisen.

8. Vorrichtung gemäss einem der Ansprüche 1 bis 7, **dadurch gekennzeichnet, dass** die Mittel für eine fluidische Verbindung wenigstens eine kommunizierende Öffnung (34) aufweisen, die in der Wandung der Vorrichtung (1; 1.1) eingerichtet ist.

9. Vorrichtung gemäss Anspruch 8, **dadurch gekennzeichnet, dass** der Hauptkanal (5) durch einen weichen Schlauch (4) gebildet ist und die Mittel für eine fluidische Verbindung ausserdem einen weichen dichten Umhüllungsschlauch (63) aufweisen, der wenigstens auf einem Teil seiner Länge den weichen Schlauch (4) umhüllt und der einen Randweg (65) um den weichen Schlauch (4) herum bildet, in den die kommunizierende Öffnung (34) mündet und durch den die angesaugte und mit dem abgezweigten Teil des Atemgases gemischte Umgebungsluft vom proximalen Ende (63B) des weichen Umhüllungsschlauchs (63) aus fliessen kann.

10. Vorrichtung gemäss Anspruch 9, **dadurch gekennzeichnet, dass** die Ansaugmittel (38) direkt auf dem weichen Schlauch (4) im Bereich seines proximalen Endes (2) montiert sind.

11. Vorrichtung gemäss einem der Ansprüche 1 bis 10, **dadurch gekennzeichnet, dass** gegenüber der distalen Öffnung (18; 51A) des Hilfskanals (8; 51) Mittel (15; 45) zum Ablenken des Strahls des Atemgases zur Achse (17; 50) des Hauptkanals (5; 44) hin vorgesehen sind und dass die Verbindungsmittel (34, 35) zwischen den Ablenkmitteln (15; 45) und dem distalen Ende (7; 47) des Hauptkanals (5; 44) angeordnet sind.

## Claims

1. A device for respiratory assistance which is tubular and forms a main channel (5; 44) to be connected via its distal portion (3; 49) to an airway of a patient such that said main channel (5; 44) connects the respiratory system of said patient to the outside, said device (1; 1.1) comprising:
- at least one auxiliary channel (8; 51) connected to a source (25) of breathable gas so as to be able to insufflate a jet of such breathable gas through at least one distal orifice (18; 51A) arranged before the distal end (7; 47) of said main channel (5; 44); and
means of fluid communication which are arranged between said distal orifice (18; 51A) of said auxiliary channel (8; 51) and said distal end (7; 47) of said main channel (5; 44),
**characterized in that**:
- said device (1; 1.1) for respiratory assistance comprises:
means of diversion (36) for diverting a volume fraction of said breathable gas intended for said auxiliary channel (8; 51), before it enters the latter; and
means of aspiration (38) which aspirate ambient air and are driven by said diverted fraction of breathable gas;
- and said means of aspiration (38) are connected to said means of communication (34) in such a way that the latter are able to convey the aspirated ambient air, mixed with said diverted fraction of breathable gas, into said main channel (5; 44).

2. The device according to claim 1, **characterized in that** it comprises means (39) of regulating said fraction of breathable gas diverted by said means of diversion (36).

3. The device according to claim 2, **characterized in that** said regulating means (39) are arranged between said means of diversion (36) and said means of aspiration (38).

4. The device according to one of claims 2 and 3, **characterized in that** said regulating means (39) comprise at least one valve.

5. The device according to one of claims 1 to 4, **characterized in that** it additionally comprises means (40) of adjusting the flow rate of diluted breathable gas issuing from said means of aspiration (38) and intended to enter said main channel (5; 44).

6. The device according to claim 5, **characterized in that** said means (40) of adjusting the flow rate are arranged between said means of aspiration (38) and said means of fluid communication (34).

7. The device according to one of claims 5 and 6, **characterized in that** said means (40) of adjusting the flow rate comprise at least one valve.

8. The device according to one of claims 1 to 7, **characterized in that** said means of fluid communication comprise at least one communication orifice (34) which is formed in the wall of said device (1; 1.1).

9. The device according to claim 8, **characterized in that** said main channel (5) being formed by a flexible tube (4), said means of fluid communication further comprise a flexible airtight sheath (63) which surrounds said flexible tube (4), at least on a portion of its length, and which forms a peripheral channel (65) around said flexible tube (4), wherein said communication orifice (34) opens into and by which aspired ambient air mixed with said diverted fraction of breathable gas is able to flow from the proximal end (63B) of said flexible sheath (63).

10. The device according to claim 9, **characterized in that** said means of aspiration (38) are directly mounted on said flexible tube (4) near to its proximal end (2).

11. The device according to one of claims 1 to 10, **characterized in that** means (15; 45) of deflecting said jet of breathable ventilation gas toward the axis (17; 50) of said main channel (5; 44) are provided opposite said distal orifice (18; 51A) of said auxiliary channel (8; 51), and **in that** said means of communication (34, 35) are arranged between said means of deflection (15; 45) and said distal end (7; 47) of said main channel (5; 44).
